# FASCICULE DE BREVET EUROPEEN

(11) **EP 2 370 140 B1**
(45) Date de publication et mention de la délivrance du brevet: **13.02.2019**
(21) Numéro de dépôt: 09801744.5
(22) Date de dépôt: 04.12.2009
(51) Int. Cl.: A61M 31/00, B65D 81/32, B65D 83/00, B65D 51/28, B65D 25/42

(54) **DISPOSITIF DE CONDITIONNEMENT ET D'ADMINISTRATION DE PRINCIPES ACTIFS EN SOLUTION HYDRO-ALCOOLIQUE**
VORRICHTUNG ZUR VERPACKUNG UND ABGABE VON WIRKSTOFFEN IN EINER HYDROALKOHOLISCHEN LÖSUNG
DEVICE FOR PACKAGING AND DELIVERING ACTIVE PRINCIPLES IN A HYDROALCOHOLIC SOLUTION

(30) Priorité: 05.12.2008 FR 0858314
(43) Date de publication de la demande: 05.10.2011
(73) Titulaire: Perovitch, Philippe, 33680 Le Temple (FR); Maury, Marc, 33160 Saint Medard en Jalles (FR)
(72) Inventeur: Perovitch, Philippe, 33680 Le Temple (FR); Maury, Marc, 33160 Saint Medard en Jalles (FR)
(74) Mandataire: CAPRI
(86) Numéro de dépôt international: PCT/FR2009/052413
(87) Numéro de publication internationale: WO 2010/063978

(56) Documents cités:
- EP-A- 0 812 775
- EP-B1- 1 339 616
- WO-A-00/53507
- US-A- 3 802 604
- US-A- 5 827 235
- US-A1- 2007 005 027
- US-A1- 2007 009 490

## Description

La présente invention concerne un dispositif de conditionnement et d'administration de principes actifs en solution hydro-aloolique.

On recherche de plus en plus à administrer les médicaments sous forme liquide, en particulier sous forme de solutions susceptibles de permettre la diffusion d'actifs par perméation sub-linguale, jugale et/ou para-gingivale, à travers les muqueuses couvrant la cavité buccale.

En effet, ce mode d'administration per-muqueux est très avantageux car il permet une biodisponibilité et une action pharmacologique systémique quasi-instantanée du fait du passage en quelques secondes du ou des principes actifs déposés au contact de la muqueuse.

Cette action rapide et ciblée qui est obtenue avec de faibles dosages de principe(s) actif(s), évite ainsi de nombreux effets secondaires liés aux métabolismes des drogues (digestif, hépatique) avant leur mise à disposition pharmacologique systémique, organique et tissulaire.

Toutefois, le conditionnement de ces formulations et leur administration aux patients posent un certain nombre de contraintes.

En particulier, il est généralement nécessaire de prévoir un conditionnement en uni-doses, facile d'utilisation et sécurisé, dont la premier paramètre de sécurité est d'éviter un surdosage dangereux.

De plus, dans le cas de solutions hydro-alcooliques à passage per-muqueux buccal, il est essentiel de préserver la stabilité de la solution, de conserver le degré d'alcool précis de la formulation et de protéger le ou les principes actifs de toute altération ou dégradation.

On connaît différents dispositifs de conditionnement uni-doses, notamment des petits flacons stériles ou des sachets pour formes buvables, à verser dans un verre après ouverture. Ces contenants ne permettent pas d'obtenir les garanties d'une bonne administration du médicament présenté en solution hydro-alcoolique avec un degré alcoolique élevé, destiné à un passage per-muqueux buccal.

On connaît également dans l'industrie du conditionnement médicamenteux notamment, le "stick" souple qui est très attractif pour son coût, sa rapidité de fabrication. De plus, ce stick permet le recours à de très nombreuses variantes de complexes de films, en matériau polymère et/ou métallique, utilisables en fonction des produits contenus dans ledit stick.

Le document US 3,802,604 A1 décrit un dispositif permettant le stockage et transport de deux substances distinctes ainsi que leur mélange avant distribution par perçage d'une membrane par un trocar situé dans le bouchon. Les deux produits sont, après mélange, distribués simultanément via le bouchon.

Le document WO 00/53507 décrit un dispositif comprenant un bouchon adaptable sur un emballage principal comprenant une substance. Le bouchon contenant une substance différente et étant muni d'un outil de ponction pour mélanger les deux substance avnt délivrance.

C'est pourquoi la présente invention vise à pallier les inconvénients de l'art antérieur en proposant un dispositif particulièrement adapté au conditionnement et à l'administration de petits volumes de principes actifs en solution hydro-alcoolique.

De plus, il se pose un autre problème dès que la solution totale à administrer comprend au moins deux éléments dont l'instabilité nécessite un mélange au seul moment de l'administration, cette composition totale n'étant pas stable dans le temps.

A cet effet, l'invention a pour objet un dispositif de conditionnement et d'administration d'une solution hydro-alcoolique tel que décrit dans la revendication 1. Des modes de réalisation avantageux sont décrits dans les revendications dépendantes. Pour améliorer la préhension et la manipulation, il est prévu d'adjoindre des moyens de délivrance de la solution contenue dans ledit stick, notamment comprenant un poussoir et des moyens de rappel.

Le dispositif selon la présente invention est maintenant décrit en détail suivant des modes de réalisation particuliers, non limitatifs, en regard des dessins annexés, dessins sur lesquels les différentes figures représentent :
- figure 1 : une vue du mode minimaliste de réalisation du dispositif de conditionnement et d'administration selon la présente invention.
- figure 2 : une vue en perspective schématique d'un premier mode de réalisation du dispositif de conditionnement et d'administration selon la présente invention, avant usage.
- figure 3 : une vue en perspective schématique d'un mode de réalisation du dispositif de conditionnement et d'administration de la figure 2, après usage.
- figure 4 : une vue en coupe transversale du dispositif de conditionnement et d'administration de la figure 2, avant usage.
- figure 5 : une vue en coupe transversale du dispositif de conditionnement et d'administration de la figure 2, en préparation avant usage.
- figure 6 : une vue en coupe transversale du dispositif de conditionnement et d'administration de la figure 2, après usage.
- figure 7 : une vue en perspective schématique d'un second mode de réalisation du dispositif de conditionnement et d'administration selon la présente invention, avant usage.
- figure 8: une vue en perspective schématique du mode de réalisation du dispositif de conditionnement et d'administration de la figure 7, après usage,
- figure 9 : une vue en coupe transversale du dispositif de conditionnement et d'administration de la figure 7, avant usage.
- figure 10 : une vue en coupe transversale du dispositif de conditionnement et d'administration de la figure 7, en préparation avant usage.
- figure 11 : une vue en coupe transversale du dispositif de conditionnement et d'administration de la figure 7, après usage.

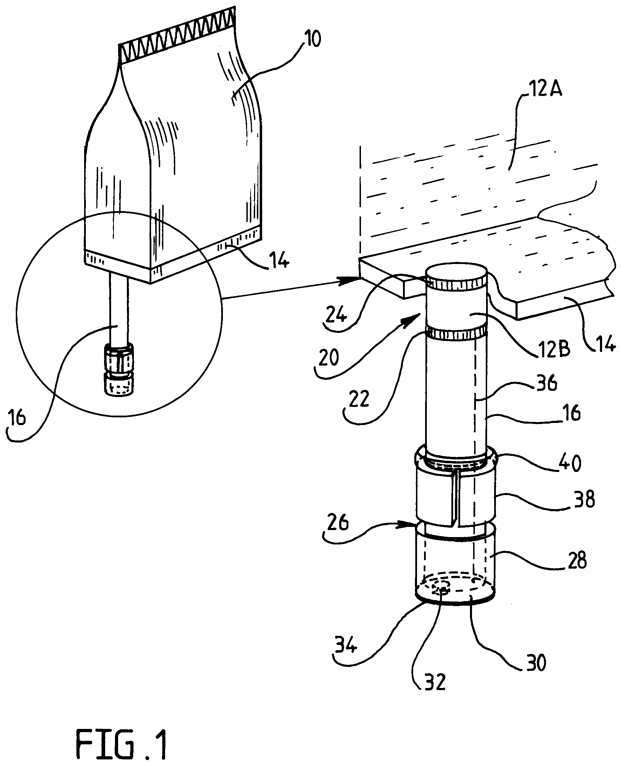

Le dispositif selon l'invention est décrit dans son mode de réalisation minimaliste en accord avec la figure 1.

Le dispositif selon l'invention comprend un contenant 10 déformable, notamment une enveloppe qui peut être en toute matière déformable, destiné à conserver une solution 12A. Préférentiellement, le contenant est réalisé en une matière qui permet de conserver cette solution 12A, mais aussi de la protéger de l'action de la lumière et d'éviter son évaporation à travers les parois.

Ce contenant 10 déformable est solidaire, de façon étanche, d'une embase 14 rigide, par exemple en matière plastique rigide.

Le dispositif comprend en outre un tube 16 d'administration de la solution 12. Ce tube 16 est avantageusement solidaire de l'embase 14 rigide. Ce tube est plus spécifiquement venu de moulage avec ladite embase 14.

Ce tube 16 est débouchant par son extrémité 18 intérieure dans le contenant 10. Il comprend, dans son volume intérieur, une chambre 20 définie par deux membranes 22 et 24 perforables et destinées à recevoir une seconde solution 12B destinée à être combinée à la première solution 12A pour former la solution 12 thérapeutique destinée à être administrée.

A son extrémité 26 extérieure, le tube 16 porte un bouchon 28, en l'occurrence un bouchon vissant.

La face 30 peut être munie d'un trou 32 de délivrance obturé par un opercule 34 d'étanchéité et de sécurité prévu pour être arraché.

Cette face 30 du bouchon, côté interne, porte également un élément en saillie formant un trocart 36 orienté vers l'intérieur.

Ainsi, deux solutions pour l'administration, soit le bouchon 28 est laissé en place et l'opercule 34 est retiré pour permettre l'administration à travers le trou 32 de délivrance, soit le bouchon 28 avec son trocart 36 est retiré pour permettre l'administration par le tube 16.

Une bague 38 de sécurité amovible est positionnée entre une butée 40 solidaire dudit tube 16 et ledit bouchon.

Le bouchon 28 peut ainsi prendre deux positions l'une avant usage dans laquelle tout est scellé et obturé et l'autre pour préparer l'usage dans laquelle la bague 38 de sécurité est d'abord retirée, le bouchon 28 vissé jusqu'à venir en appui sur la butée 40, le trocart 36 assurant alors la perforation des deux membranes 22 et 24 perforables et la libération de la solution 12B contenue dans la chambre 20.

De ce fait, les deux solutions 12A et 12B peuvent se mélanger et la solution 12 hydro-alcoolique thérapeutique est prête à l'usage et à être administrée.

Le fonctionnement du dispositif de conditionnement et d'administration selon la présente invention dans sa version minimaliste est maintenant décrit en détail en regard de la figure 1.

L'utilisateur retire la bague 38 de sécurité et visse le bouchon 28 à fond de façon que le trocart 36 vienne perforer les membranes 22 et 24.

La composition 12B, libérée de la chambre 20, se mélange avec la composition 12A du contenant 10.

Soit l'utilisateur retire l'opercule 34 et libère le trou 32 pour permettre la délivrance de la composition thérapeutique dans la cavité buccale à travers le tube 16, soit l'utilisateur retire le bouchon 28 et son trocart.

L'utilisateur place l'extrémité du tube 16 par son extrémité 26 extérieure sous la langue ou en toute zone anatomique de la cavité buccale.

L'utilisateur peut ensuite presser sur le contenant déformable et/ou peut aspirer pour assurer la délivrance complète de l'ensemble de la composition 12 thérapeutique.

Ceci s'effectue d'autant plus facilement que la zone supérieure se trouve emplie d'air, ladite pression d'air assurant un effet de chasse de la composition.

Ce mode de réalisation permet de répondre aux différentes contraintes imposées.

On résout le problème des compositions instables de produits puisque ceux ci sont mélangés au seul moment de l'utilisation sans risquer d'être mélangés avant et mis en attente. En effet, dès que le contenant est prêt à être utilisé, il ne peut plus être posé ou mis en attente, il doit être administré.

Cet agencement répond également aux contraintes imposées par l'industrie tant du point de vue fabrication en grand nombre que du point de vue des coûts. Ce contenant permet aussi de garantir la qualité pharmaceutique très particulière des solutions hydro-alcooliques, vecteurs des principes actifs à administrer par voie buccale per-muqueuse.

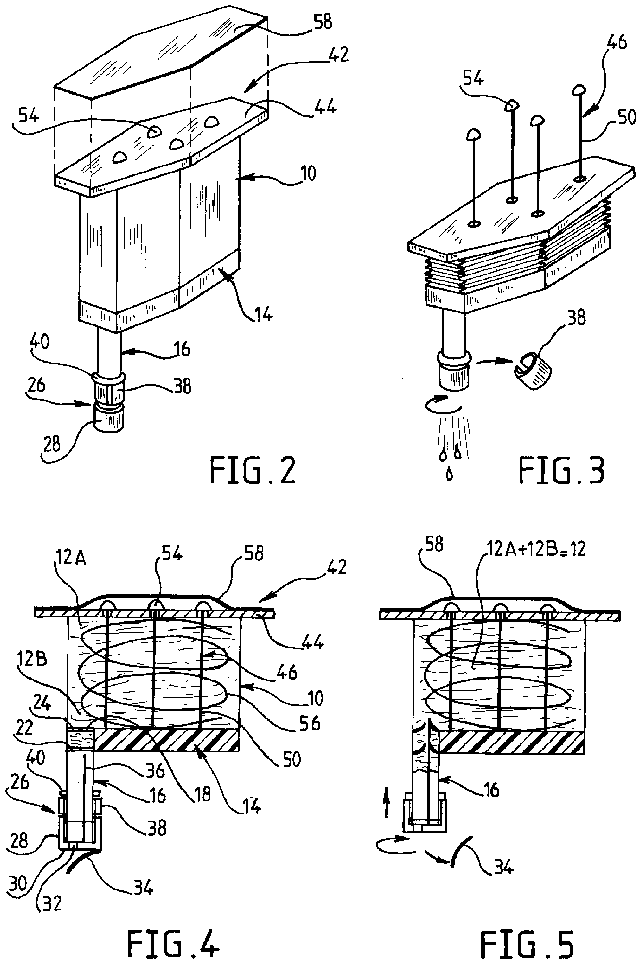

Selon un mode de réalisation décrit en regard des figures 2 à 5 indifféremment, le dispositif selon l'invention comprend en outre des moyens 42 de délivrance de la solution 12 à administrer.

Ces moyens 42 de délivrance comprennent une platine 44 de manoeuvre, des moyens 46 de guidage et des moyens 48 de rappel élastique.

La platine 44 de manoeuvre est solidarisée au contenant 10 déformable, en regard de l'embase 14. Cette platine 44 est apte à prendre au moins deux positions extrêmes, l'une dans laquelle la platine 44 est éloignée de l'embase 14, le contenant 10 déformable étant plein de la solution 12A et d'air éventuellement, avant usage et l'autre dans laquelle la platine 44 est plaquée contre ladite embase, la solution 12 ayant été expulsée, après usage. Les moyens 46 de guidage comprennent, dans le mode de réalisation représenté, au moins deux tiges 50, en l'occurrence 4 tiges. Ces tiges 50 passent à travers des trous 52 ménagés dans la platine 44 de manoeuvre de sorte à permettre le guidage de la dite platine durant son déplacement en translation pour passer de la première position extrême à l'autre position extrême. Les extrémités de ces tiges 50 de guidage portent des têtes 54 évitant le désengagement de cette platine hors de ces tiges de guidage.

Les moyens 48 de rappel élastique comprennent dans ce mode de réalisation, un ressort 56 du type hélicoïdal, avantageusement en matière plastique. Le diamètre du fil constituant le ressort est très fin et de préférence à enroulement conique de façon à pouvoir s'aplatir en ne laissant comme épaisseur morte que le diamètre du fil une fois comprimé.

L'agencement décrit est complété par une vignette 58 de sécurité qui est prévue pour être disposée sur la platine 44 de manoeuvre de façon à permettre un verrouillage de ladite platine, évitant tout déplacement intempestif. En effet, la platine se trouve immobilisée du fait que les têtes 54 ne peuvent pas faire saillie à travers la platine 44. De façon avantageuse, cette vignette est adhésive.

Dès que la vignette 58 de sécurité est retirée, la platine est libre et peut être manoeuvrée en translation.

On note dans cet agencement que la platine 44 de manoeuvre est débordante pour permettre une prise tri digitale. En effet le pouce Po est prévu pour être en appui sur l'embase 14, calé par le tube 16 tandis que l'index In et le majeur Ma trouvent un positionnement naturel sur les extrémités de cette platine. Ces positions sont représentées en In et Ma sur les figures 2 et 5.

Pour donner un ordre de grandeur, selon un mode de réalisation particulier de l'invention, pour un volume de composition à administrer compris entre 0,5 et 2ml, le dispositif présente une hauteur comprise entre 3,0 et 8,0 cm, une largeur comprise entre 1,5 et 5 cm et l'embase 18 présente une hauteur comprise entre 2,0 et 5,0 cm et une largeur comprise entre 1,0 et 3 cm.

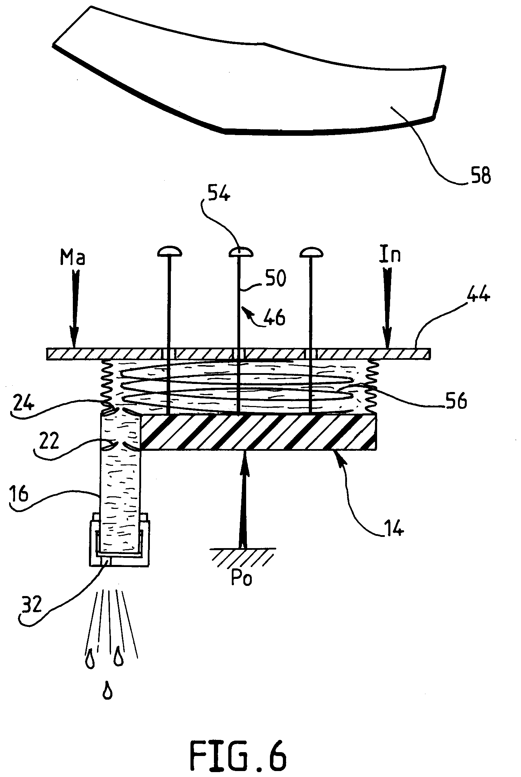

Dans le mode de réalisation perfectionné des figures 2 à 6, le dispositif fonctionne de la façon qui va maintenant être décrite.

L'enveloppe 10 contient la première solution 12A et le ressort 56 des moyens de rappel élastique assure un maintien en forme, tendue de cette enveloppe.

La vignette 58 de sécurité assure la garantie et interdit tout mouvement de la platine 44 de manoeuvre par rapport aux tiges 50 des moyens 46 de guidage. La solution est ainsi conservée sans altération du contenu et sans risque d'effraction accidentelle.

La seconde solution 12B est contenue et préservée dans la chambre 20 définie par les membranes 22 et 24 dans le tube 16.

La mise en oeuvre du dispositif de conditionnement et d'administration selon la présente invention consiste en la série d'étapes qui sont maintenant décrites. L'utilisateur retire la bague 38 de sécurité et visse le bouchon 28.

Le vissage a pour effet de perforer les deux membranes 22 et 24 du fait du déplacement du trocart 36, ce qui libère la seconde solution 12B qui peut être le principe actif. Cette seconde solution 12B se mélange alors avec la première solution 12A présente dans le contenant 10 déformable, qui peut être l'excipient. On note qu'il n'y a aucun risque d'écoulement, même en tenant le dispositif avec le tube 16 vers le bas d'une part puisque les moyens 48 de rappel élastique et les forces de capillarité assurent la retenue de la solution dans le contenant 10 déformable et d'autre part le bouchon et/ou l'opercule 34 assurent l'herméticité. Dès que l'utilisateur souhaite s'administrer la solution 12 thérapeutique obtenue par le mélange des solutions 12A et 12B, en sub-lingual, en jugal ou en para gingival par exemple, il retire la vignette 58 de sécurité, assure une prise tri digitale avec un appui du pouce Po sur l'embase 14 et en opposition un appui de l'index In et du majeur Ma sur la platine 44 de manoeuvre. Le tube est placé par son extrémité 26 extérieure sous la langue ou dans les zones anatomiques concernées de la cavité buccale.

Le mouvement relatif de la platine 44 de manoeuvre par rapport à l'embase 14, à l'encontre de l'effort de rappel des moyens de rappel élastique, qui reste extrêmement limité comparé à la puissance des doigts, assure un flux de chasse de la solution 12 thérapeutique et la propulse hors du dispositif, à travers le trou 32.

La zone supérieure emplie d'air facilite cette expulsion par un effet de chasse du volume liquide.

Durant cette opération, le contenant 10 déformable se déforme proportionnellement.

Enfin, l'utilisateur peut éventuellement exercer, en fin d'administration une légère aspiration, lèvres fermées autour du tube, pour compléter l'extraction de toute solution résiduelle dans le contenant déformable.

On note que les moyens de guidage permettent un déplacement rectiligne en translation de la platine 44 de manoeuvre.

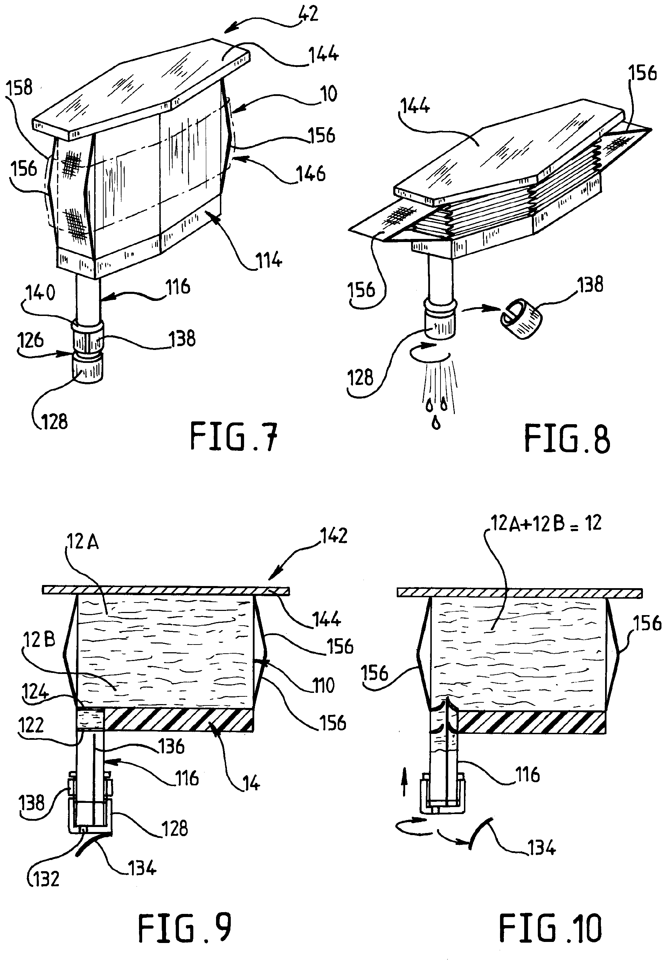

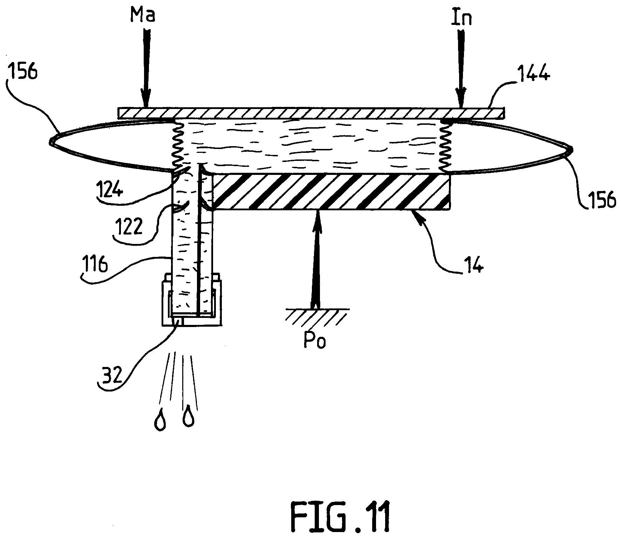

Sur les figures 7 à 11, on a représenté une variante de réalisation, la modification majeure portant sur l'agencement des moyens de rappel élastique. Afin de permettre une lecture simple, les éléments identiques ou assurant la même fonction portent les mêmes références que celles des figures 2 à 6, augmentées de 100.

Dans ce mode de réalisation, les moyens 146 de guidage et les moyens 148 de rappel élastique qui devraient être référencés, sont combinés et référencés uniquement 146.

En effet, le ressort 56 de type hélicoïdal est remplacé par des lames 156 flexibles avec amorce de pliage. Cette amorce de pliage est par exemple une légère courbure vers l'extérieur. Ces lames ont donc une largeur très supérieure à l'épaisseur.

Dans le cas présent, il est prévu deux lames 156 pour assurer la stabilité du mouvement.

Les moyens 48 de guidage du premier mode de réalisation sont supprimés au profit des moyens 148 de guidage constitués par les lames 156 elles-mêmes. Ces lames ont une largeur suffisante pour assurer un guidage en translation de la platine 144 de manoeuvre par rapport à l'embase 114.

Dans ce mode de réalisation, il faut aussi prévoir une vignette 158 de sécurité. Cette fois ci, elle est disposée autour des lames, dans un plan médian, comme un collier afin d'interdire toute courbure intempestive des lames.

Dans ce cas, l'utilisateur procède de la même façon aux opérations de rotation du bouchon 128 pour perforer les membranes 122 et 124 et assurer le mélange des solutions 12A et 12B pour obtenir la solution 12 thérapeutique définitive.

Il retire ensuite la vignette 158 de sécurité.

Le dispositif est prêt à fonctionner.

Il suffit de retirer l'opercule 134 éventuel pour assurer la libération du trou 132 ou de retirer le bouchon 128 et son trocart 136, de positionner le tube 116 dans la bouche en sub lingual, jugal ou para gingival, de positionner les doigts en tri digital et d'exercer une pression sur la platine de manoeuvre 144, à l'encontre de l'effort de rappel élastique des lames 156. Cet effort reste faible comparé à la puissance disponible avec des doigts.

Les lames 156 se plient en se courbant et le contenant 110 déformable se plie proportionnellement en assurant un flux de chasse de la solution 12 et la propulsant hors du dispositif, à travers le trou 132 ou l'extrémité du tube 116. Enfin, l'utilisateur peut éventuellement exercer une légère aspiration, lèvres fermées autour du tube, pour compléter l'extraction de toute solution résiduelle dans le contenant déformable.

On note que les lames 156 assurent un déplacement rectiligne en translation de la platine 144 de manoeuvre qui vient en regard de l'embase 114.

Il est à noter que la section proposée du contenant 10, 110 déformable du type losange peut être à section ovale, ou circulaire sans changer la présente invention.

Le tube 16, 116 porte un bouchon 28, 128 à vis mais un bouchon à pousser pourrait être un total équivalent technique tombant sous le coup de la présente invention.

La description ci-avant est relative à un agencement donné dans lequel il n'y a que deux membranes, deux solutions mais il est bien sûr possible d'en augmenter le nombre tout en restant dans le cadre de la même invention.

De même, pour une bonne compréhension, les solutions ont été décrites comme liquides mais il en serait de même avec le recours à un des composés sous forme de poudres, de pâte, de gel ou de granules.

La composition thérapeutique finale hydro-alcoolique est par contre, elle, toujours liquide.

De la même façon, il a été défini une chambre dans le tube à l'aide de deux membranes mais le tube lui-même peut constituer la chambre et dans ce cas, une seule membrane est nécessaire.

On peut aussi envisager des variantes qui seront considérées comme des équivalents techniques. Ainsi au lieu de prévoir un mouvement de translation d'une platine contre une embase, il est possible de prévoir un mouvement de pince, embase et platine étant liées en un point d'articulation en logeant le contenant déformable dans l'angle formé par les embase et platine de sorte à permettre un écrasement dudit contenant par pincement en fermant l'angle de la pince.

## Revendications

1. Dispositif de conditionnement et d'administration d'une solution (12) hydro-alcoolique à passage per-muqueux, comprenant un contenant déformable (10, 110) sous forme d'enveloppe ou de stick souple destiné à recevoir au moins une première partie (12A) de la solution, une embase rigide (14,114) solidaire dudit contenant, un tube (16,116) d'administration solidaire de ladite embase, apte à recevoir au moins une seconde partie (12B) de la solution et comprenant un bouchon (28,128), **caractérisé en ce que** le tube (16,116) comprend une chambre destinée à contenir ladite au moins une seconde solution (12B), délimitée par deux membranes (22,24;122;124) et **en ce que** le bouchon (28, 128) est mobile en translation entre deux positions extrêmes et comprend un trocart (36, 136) destiné à venir percer les deux dites membranes dans l'une des positions extrêmes, ledit dispositif comprenant une platine rigide (44,144) de manoeuvre solidaire du contenant (10,110) déformable, apte à prendre au moins deux positions extrêmes, l'une dans laquelle la platine (44,144) est éloignée de l'embase (14,114), le contenant (10,110) déformable étant plein de la solution (12A), avant usage et l'autre dans laquelle la platine (44,144) est plaquée contre ladite embase, la solution (12) ayant été expulsée, ledit dispositif comprenant en outre des moyens (46,146) de guidage et des moyens (48,148) de rappel élastique, le bouchon (28, 128) comprenant un trou (32,132) de délivrance de la solution (12) et un opercule (34, 134).

2. Dispositif de conditionnement et d'administration d'une solution (12) hydro-alcoolique à passage per-muqueux selon la revendication 1, dans lequel les moyens (46) de guidage comprennent au moins une tige (50) solidaire de l'embase (14), montée coulissante à travers la platine (44) de manoeuvre et munie d'une tête (54) d'arrêt.

3. Dispositif de conditionnement et d'administration d'une solution (12) hydro-alcoolique à passage per-muqueux selon la revendication 2, dans lequel les moyens (48) de rappel élastique comprennent un ressort (56) de rappel hélicoïdal disposé dans le contenant déformable.

4. Dispositif de conditionnement et d'administration d'une solution (12) hydro-alcoolique à passage per-muqueux selon la revendication 1, dans lequel que les moyens (146) de guidage et les moyens (148) de rappel élastique comprennent au moins deux lames (156).

5. Dispositif de conditionnement et d'administration d'une solution (12) hydro-alcoolique à passage per-muqueux selon la revendication 4, dans lequel chaque lame (156) comprend une amorce de pliage sous forme d'une courbure vers l'extérieur.

6. Dispositif de conditionnement et d'administration d'une solution (12) hydro-alcoolique à passage per-muqueux selon l'une quelconque des revendications 1 à 5, comprenant une vignette (58,158) de sécurité.

7. Dispositif de conditionnement et d'administration d'une solution (12) hydro-alcoolique à passage per-muqueux selon l'une quelconque des revendications précédentes, présentant un volume compris entre 0,5 et 2ml, une hauteur comprise entre 3,0 et 8,0 cm, une largeur comprise entre 1,5 et 5,0 cm et l'embase (18,118) présente une hauteur comprise entre 2,0 et 4,0 cm et une largeur comprise entre 1,0 et 5,0 cm pour une administration sub linguale, jugale ou para gingivale.

## Patentansprüche

1. Vorrichtung zur Aufbereitung und Verabreichung einer hydroalkoholischen Lösung (12) zur permukösen Aufnahme, umfassend einen verformbaren Behälter (10, 110) in Form einer Umhüllung oder eines flexiblen Stifts zur Aufnahme mindestens eines ersten Teils (12A) der Lösung, eine starre Grundfläche (14, 114) in einem Stück mit dem Behälter, ein Verabreichungsrohr (16, 116) in einem Stück mit der Grundfläche, das dazu geeignet ist, mindestens einen zweiten Teil (12B) der Lösung aufzunehmen, und das einen Pfropfen (28, 128) umfasst, **dadurch gekennzeichnet, dass** das Rohr (16, 116) eine Kammer umfasst, die dazu gedacht ist, die mindestens eine zweite Lösung (12B) zu enthalten, und die durch zwei Membranen (22, 24; 122; 124) abgegrenzt ist, und dass der Pfropfen (28, 128) zwischen zwei Endpositionen verschieblich ist und einen Trokar (36, 136) umfasst, der dazu gedacht ist, in einer der Endpositionen die zwei Membranen zu durchstoßen, wobei die Vorrichtung eine starre Platte (44, 144) umfasst, die mit dem verformbaren Behälter (10, 110) zusammen betätigbar und dazu geeignet ist, mindestens zwei Endpositionen einzunehmen, und zwar eine vor der Benutzung, in der die Platte (44, 144) von der Grundfläche (14, 114) entfernt ist, wobei der verformbare Behälter (10,110) mit der Lösung (12A) gefüllt ist, und eine andere, in der die Platte (44, 144) gegen die Grundfläche gepresst wird, wobei die Vorrichtung, wenn die Lösung (12) ausgetrieben worden ist, des Weiteren Mittel (46, 146) zum Führen und Mittel (48, 148) zum elastischen Rückführen umfasst, wobei der Pfropfen (28, 128) ein Loch (32, 132) zur Ausgabe der Lösung (12) und ein Verschlussorgan (34, 134) umfasst.

2. Vorrichtung zur Aufbereitung und Verabreichung einer hydroalkoholischen Lösung (12) zur permukösen Aufnahme nach Anspruch 1, wobei die Mittel (46) zum Führen mindestens eine Stange (50) in einem Stück mit der Grundfläche (14) umfassen, die durch die Betätigungsplatte (44) gleitend montiert und mit einem Anschlagkopf (54) versehen ist.

3. Vorrichtung zur Aufbereitung und Verabreichung einer hydroalkoholischen Lösung (12) zur permukösen Aufnahme nach Anspruch 2, wobei die Mittel (48) zum elastischen Rückführen eine schraubenförmige Rückstellfeder (56) umfassen, die in dem verformbaren Behälter angeordnet ist.

4. Vorrichtung zur Aufbereitung und Verabreichung einer hydroalkoholischen Lösung (12) zur permukösen Aufnahme nach Anspruch 1, wobei die Mittel (146) zum Führen und die Mittel (148) zum elastischen Rückführen mindestens zwei Lamellen (156) umfassen.

5. Vorrichtung zur Aufbereitung und Verabreichung einer hydroalkoholischen Lösung (12) zur permukösen Aufnahme nach Anspruch 4, wobei jede Lamelle (156) ein Faltband in Form einer Krümmung nach außen umfasst.

6. Vorrichtung zur Aufbereitung und Verabreichung einer hydroalkoholischen Lösung (12) zur permukösen Aufnahme nach einem der Ansprüche 1 bis 5, umfassend eine Sicherheitsbanderole (58, 158).

7. Vorrichtung zur Aufbereitung und Verabreichung einer hydroalkoholischen Lösung (12) zur permukösen Aufnahme nach einem der vorhergehenden Ansprüche, aufweisend ein Volumen zwischen 0,5 und 0,2 ml, eine Höhe zwischen 3,0 und 8,0 cm, eine Breite zwischen 1,5 und 5,0 cm, und wobei die Grundfläche (18, 118) eine Höhe zwischen 2,0 und 4,0 cm und eine Breite zwischen 1,0 und 5,0 cm für eine sublinguale oder paragingivale Verabreichung oder eine Verabreichung über die Wange umfasst.

## Claims

1. A packaging and administering device for administering a hydroalcoholic solution (12) by passing through a mucous membrane, the device comprising a deformable container (10, 110) in the form of a pouch or of a flexible stick for receiving at least one first fraction (12A) of the solution, a rigid base (14, 114) that is secured to said container, and an administering tube (16, 116) that is secured to said base, that is suitable for receiving at least one second fraction (12B) of the solution, and that includes a cap (28, 128), the device being **characterized in that** the tube (16, 116) includes a chamber for containing said at least one second solution (12B) and that is defined by two membranes (22, 24; 122; 124), and **in that** the cap (28, 128) is movable in translation between two extreme positions and includes a needle (36, 136) for coming to pierce the two said membranes in one of the extreme positions, said device including a movable rigid plate (44,144) that is secured to the deformable container (10, 110), said plate being capable of taking up at least two extreme positions, one in which the plate (44, 144) is at a distance from the base (14, 114), prior to use, the deformable container (10, 110) being full of solution (12A), and the other in which the plate (44, 144) is pressed against said base, after the solution (12) has been expelled, said device further including guide means (46, 146) and resilient return means (48, 148), the cap (28, 128) including a delivery hole (32, 132) for delivering the solution (12), and a film (34, 134).

2. A packaging and administering device for administering a hydroalcoholic solution (12) by passing through a mucous membrane according to claim 1, in which the guide means (46) comprise at least one rod (50) that is secured to the base (14), that is mounted to slide through the movable plate (44), and that is provided with a stopper head (54).

3. A packaging and administering device for administering a hydroalcoholic solution (12) by passing through a mucous membrane according to claim 2, in which the resilient return means (48) include a helical return spring (56) that is disposed in the deformable container.

4. A packaging and administering device for administering a hydroalcoholic solution (12) by passing through a mucous membrane according to claim 1, in which the guide means (146) and the resilient return means (148) comprise at least two blades (156).

5. A packaging and administering device for administering a hydroalcoholic solution (12) by passing through a mucous membrane according to claim 4, in which each blade (156) includes a fold starter in the form of an outward bend.

6. A packaging and administering device for administering a hydroalcoholic solution (12) by passing through a mucous membrane according to any one of claims 2 to 5, including a safety label (58,158).

7. A packaging and administering device for administering a hydroalcoholic solution (12) by passing through a mucous membrane according to any preceding claim, presenting a volume lying in the range 0.5 mL to 2 mL, a height lying in the range 3.0 cm to 8.0 cm, a width lying in the range 1.5 cm to 5.0 cm, and the base (14, 114) presents a height lying in the range 2.0 cm to 4.0 cm, and a width lying in the range 1.0 cm to 5.0 cm, for administration under the tongue, via the cheeks, or via the gums.
